(19) Europäisches Patentamt / European Patent Office / Office européen des brevets

(11) **EP 3 377 881 B1**

(12) **EUROPEAN PATENT SPECIFICATION**

(45) Date of publication and mention
of the grant of the patent:
**04.01.2023 Bulletin 2023/01**

(21) Application number: **16791009.0**

(22) Date of filing: **03.11.2016**

(51) International Patent Classification (IPC):
*G01N 25/18* (2006.01)     *G01N 33/487* (2006.01)
*G01N 33/544* (2006.01)     *G01N 33/569* (2006.01)
*B01J 20/26* (2006.01)

(52) Cooperative Patent Classification (CPC):
**G01N 25/18; B01J 20/268; G01N 33/487;
G01N 33/543; G01N 33/544; G01N 33/56911;**
G01N 33/531; G01N 2600/00

(86) International application number:
**PCT/EP2016/076571**

(87) International publication number:
**WO 2017/084885 (26.05.2017 Gazette 2017/21)**

(54) **DEVICES AND METHODS FOR DETECTING ANALYTES USING THERMAL WAVES**

VORRICHTUNG UND METHODEN ZUM NACHWEIS VON ANALYTEN MITTELS THERMISCHER
WELLEN

DISPOSITIF ET PROCÉDÉS DE DÉTECTION D'ANALYTES AU MOYEN D'ONDES THERMIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.11.2015 EP 15194837
29.03.2016 EP 16162550
29.03.2016 EP 16162685
29.03.2016 US 201662314461 P
11.04.2016 EP 16164636
11.04.2016 US 201615095636**

(43) Date of publication of application:
**26.09.2018 Bulletin 2018/39**

(73) Proprietors:
• **Maastricht University
6211 LK Maastricht (NL)**
• **Academisch Ziekenhuis Maastricht
6229 HX Maastricht (NL)**

(72) Inventors:
• **GRINSVEN, VAN, Bart Robert Nicolaas
6200 MD Maastricht (NL)**
• **CLEIJ, Thomas Jan
6200 MD Maastricht (NL)**

(74) Representative: **Algemeen Octrooi- en
Merkenbureau B.V.
P.O. Box 645
5600 AP Eindhoven (NL)**

(56) References cited:
**EP-A1- 2 772 753     JP-A- 2005 345 385
US-A- 6 161 028**

• **BART VAN GRINSVEN ET AL: "The Heat-Transfer
Method: A Versatile Low-Cost, Label-Free, Fast,
and User-Friendly Readout Platform for
Biosensor Applications", ACS APPLIED
MATERIALS AND INTERFACES, vol. 6, no. 16, 8
August 2014 (2014-08-08) , pages 13309-13318,
XP055252140, US ISSN: 1944-8244, DOI:
10.1021/am503667s**
• **KAROLIEN BERS ET AL: "Supporting
Information - HTM-based cell detection at trace
levels using a progressive enrichment approach
with highly selective cell-binding surface
imprints", LANGMUIR, vol. 30, no. 12, 1 April 2014
(2014-04-01) , pages 1-4, XP055307588,**

**(Cont. next page)**

EP 3 377 881 B1

- **DICKERT F L ET AL: "Synthetic receptors as sensor coatings for molecules and living cells", THE ANALYST, R S C PUBLICATIONS, GB, vol. 126, no. 6, 1 June 2001 (2001-06-01), pages 766-771, XP002436466, ISSN: 0003-2654, DOI: 10.1039/B009893K**
- **KAROLIEN BERS ET AL: "Heat-Transfer Resistance Measurement Method (HTM)-Based Cell Detection at Trace Levels Using a Progressive Enrichment Approach with Highly Selective Cell-Binding Surface Imprints", LANGMUIR, vol. 30, no. 12, 1 April 2014 (2014-04-01) , pages 3631-3639, XP055307366, US ISSN: 0743-7463, DOI: 10.1021/la5001232**

**Description**

TECHNICAL FIELD

Embodiments of the present disclosure relate generally to devices and methods of detecting analytes using polymer materials, such as over a heat sink configured to produce a thermal wave.

BACKGROUND

Molecularly imprinted polymers (MIPs) can be used for detecting chemical substances in complex mixtures. In modern research, these polymers are of increasing interest for bioanalytical applications. Advantages of using these MIPs include easy and cheap production; mechanical, chemical, and thermal stability; reusability; and long shelf life. In recent years, the concept of molecular imprinting has been extended to surface imprinting of thin polymer films with micrometer-sized cells to create so-called "surface imprinted polymers" (SIPs) for the detection of proteins, glycoproteins, plant viruses, human viruses, bacteria, pollen, yeast cells, and even mammalian red blood cells. SIPs are polymeric materials with indentations at the surface, with a form and function matching part of a desired target. SIPs are suitable for bonding with larger objects (e.g., cells, bacteria, *etc.),* which do not diffuse quickly through pores of an MIP. Imprinting may occur after polymerization by softening the polymer. The detection of cells using biosensors described in literature is conventionally done by gravimetric detection, electronic read-out platforms or micro-fluidic techniques. However, these techniques are often time-consuming, provide difficulties for analysis, or require expensive equipment.

For example, temperature resistance of substrates having MIPs attached thereto based on the concentration of analytes is described in U.S. Patent Application Publication 2014/0011198 A1, "Heat-Transfer Resistance Based Analysis Bioparticles," published January 9, 2014.

Other prior art disclosures describing techniques for detecting analytes using the heat transfer method are EP2772753 and the technical paper "The Heat-Transfer Method: A Versatile Low-Cost, Label-Free, Fast, and User-Friendly Readout Platform for Biosensor Applications", Bart van Grinsven et.al. ACS APPLIED MATERIALS AND INTERFACES, vol. 6, no. 16, 8 August 2014.

US6161028 discloses a method of determining an analyte concentration of a test sample by introducing a temperature gradient and measuring infrared radiation at selected analyte absorbance peak and reference wavelengths.

JP2005-345385 discloses a measuring instrument capable of measuring various thermal characteristics of a sample such as heat conductivity, heat insulating property, etc.

A low-cost sensor platform providing the capability to differentiate between cells with slight differences in shape, size, and functionalities in functional groups on their surface would be a valuable tool for modern research and industry.

DISCLOSURE

According to the invention, a device for detecting an analyte as described in claim 1 is provided.

In another aspect of the invention, a method for detecting an analyte as described in claim is provided.

In yet a further aspect of the invention, a method of forming the above device for detecting an analyte as described in claim 20 is provided.

BRIEF DESCRIPTION OF THE DRAWINGS

FIG. 1 is a simplified schematic diagram showing a device for detecting an analyte;

FIG. 2 is a simplified schematic representation showing how a thermal wave may travel in the device of FIG. 1;

FIG. 3 is a simplified schematic diagram showing another device for detecting an analyte;

FIG. 4 is a graph showing binding isotherms for dopamine as measured according to an example not forming part of the present invention;

FIG. 5 is a graph showing calibration curves for dopamine as measured according to an example not forming part of the present invention;

FIG. 6 is a graph showing changes in temperature as measured according to an example not forming part of the present invention;

FIG. 7 is a graph showing time-dependent values of thermal resistance as measured according to an example not forming part of the present invention;

FIG. 8 is a graph showing the thermal resistance data of FIG. 7 in the form of a dose-response curve;

FIG. 9 is a graph showing a thermal wave generated according to an embodiment of the invention;

FIG. 10 is a graph showing thermal waves measured after passing through a substrate according to an embodiment of the invention;

FIG. 11 is a graph showing the phase shift of the thermal waves shown in FIG. 10 as measured according to an embodiment of the invention;

FIG. 12 is a graph showing thermal waves measured after passing through a substrate according to an embodiment of the invention;

FIG. 13 is a graph showing the phase shift of thermal waves shown in FIG. 12 as measured according to an embodiment of the invention.

## MODE(S) FOR CARRYING OUT THE INVENTION

The illustrations presented herein are not actual views of any particular device or method, but are merely idealized representations employed to describe example embodiments of the present invention.

Elements common between figures may retain the same numerical designation.

As used herein, the terms "template molecule" and "template bacteria" respectively refer to molecules or bacteria used to form a molecularly imprinted polymer (MIP) or surface imprinted polymer (SIP). Such MIPs or SIPs can then detect "target molecules" or "binding partners," which have functionality corresponding to the template molecules used to form the MIP or SIP.

As used herein, the term "may" encompasses the word "can," and the term "may be" encompasses the words "is" or "are," depending on context. Furthermore, presence of the word "may" is intended to indicate options for practicing or implementing embodiments of the disclosure, without limitation.

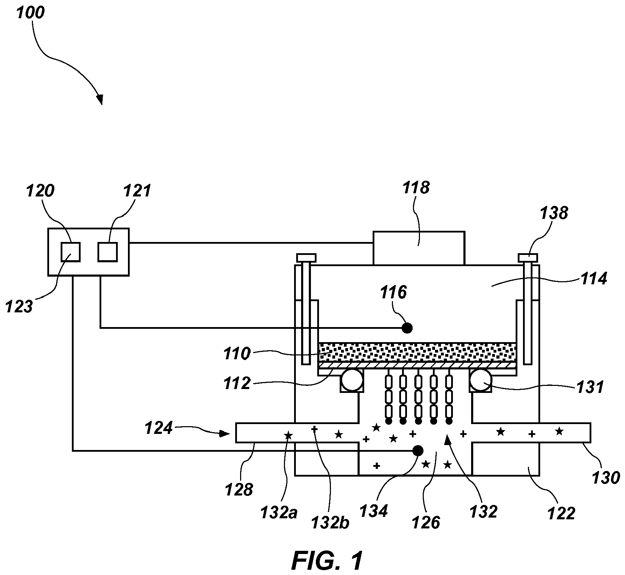

FIG. 1 is a simplified schematic diagram showing a device 100 for detecting an analyte. In some embodiments, the device 100 may be configured to detect a target molecule, a nucleic acid such as DNA and/or RNA, single-nucleotide polymorphisms (SNPs) in DNA and/or RNA, small molecules, proteins, bacteria, etc.

The device 100 includes a substrate 110 having a polymer material 112 located over a surface thereof. For example, the polymer material 112 may be formed or disposed over a generally planar surface of the substrate 110, and another, opposite generally planar surface of the substrate 110 may be free of the polymer material 112. In some embodiments, the substrate 110 may include a metal (e.g., aluminum), an alloy, a semiconductor (e.g., silicon, doped diamond, etc.), an electrically insulating material (e.g., undoped diamond). The polymer material 112 includes any material for which a heat transfer property varies based on an amount of the analyte bound thereto. For example, the thermal conductivity, thermal diffusivity, heat capacity, or another property of the polymer material 112 may vary with concentration of the analyte on the surface thereof.

In some embodiments, the polymer material 112 may include an imprinted polymer, such as a molecularly imprinted polymer (MIP) or a surface imprinted polymer (SIP). MIPs and SIPs may also be referred to in the art as "plastic" antibodies. MIPs typically possess a high affinity for a specific binding partner, so that when such binding partners are contacted with the MIP, the molecules bind with the MIP. MIPs are synthetic receptors that contain nanocavities with high affinity for their respective target molecules. Imprinting (i.e., formation of the nanocavities) is often part of the polymerization process. MIPs are able to specifically bind targets, including bacteria, varying from small ions to large cells in complex matrices. Binding of molecules to the MIP may alter some properties of the MIP, such as thermal properties, mechanical properties, electrical properties, etc. The altered property of an MIP may, therefore, be used to detect a presence of such molecules at relatively low concentrations. MIPs are described in, for example, U.S. Patent Application Publication 2009/0281272 A1, "Monodisperse Molecularly Imprinted Polymer Beads," published November 12, 2009.

Similarly, SIPs typically possess a high affinity for a specific binding partner, but may typically bind to relatively larger objects (e.g., cells, bacteria, etc.) that do not diffuse quickly through pores of an MIP. SIPs may be polymer materials formed over a surface, then imprinted after polymerization by softening the polymer.

In certain embodiments, the polymer material 112 may include DNA, RNA, proteins, or portions or analogs thereof. For example, the device 100 may include a substrate 110 (e.g., a diamond surface) functionalized with a polymer material 112 such as DNA, RNA, a protein, a polypeptide, a nucleic acid polymer, a probe, or a portion or analog thereof (e.g., complementary DNA, antibodies, etc.). The polymer material 112 may be formulated to possess a high affinity for a specific binding partner, so that when such binding partners are contacted with the surface of the substrate 110, the molecules bind with the polymer material 112. The polymer material 112 may also bind to analogues of the binding partner (e.g., a material having similar functionality as the binding partner), though not necessarily with the same affinity as binding with the binding partner itself. In some embodiments, the polymer material 112 may include at least about seven (7) repeating units, such as ten (10) repeating units or more.

In some embodiments, the polymer material 112 may include a material screen-printed onto the substrate 110. Screen-printed materials may be manufactured efficiently and in mass quantities, with relatively high uniformity in comparison with other materials.

The device 100 further includes a heat sink 114 thermally coupled to a surface of the substrate 110 opposite the polymer material 112. Though referred to as a heat "sink" for the sake of simplicity, the heat sink 114 may be configured to provide heat to or remove heat from the substrate 110 and, so, may also be characterized as a heat transfer element 114. The heat sink or heat transfer element 114 may be a material having a high thermal conductivity, such as a transition metal (*e.g.*, copper, silver, *etc.*) or an alloy or mixture thereof. The heat sink 114 may be thermally coupled to a temperature sensor 116 (e.g., a thermocouple or another device) configured to detect a temperature of the heat sink 114. According to the invention, the heat sink or heat transfer element 114 is thermally coupled to a temperature modification device 118 and may be configured to maintain the temperature of the heat sink 114. The temperature modification device 118 may include, for example, a thermoelectric device, a heat exchanger, a fan, a resistance heater, *etc.* The temperature sensor 116 may be a resistor having a resistance that varies with temperature. If the properties of the heat sink 114 are known (e.g., if a relationship between a control signal to the modification device 118 and the temperature of the heat sink 114 is well characterized), the temperature sensor 116 may be omitted. In some embodiments, the temperature sensor 116 may be integral to the temperature modification device 118. For example, the internal resistance of the temperature modification device 118 itself may be measured to determine its temperature.

The temperature sensor 116 and the temperature modification device 118 may be connected to a controller 121 configured (*i.e.,* programmed) to control the temperature modification device 118 to cause the heat sink 114 to produce a thermal wave emanating from the heat sink 114 and through the substrate 110 (including the polymer material 112 thereon). For example, the controller 121 and a processor 123 may be incorporated into a computer 120 (*e.g.,* the controller 121 may be an input-output card configured to receive and provide electrical signals, and may be configured to receive signals from the processor 123). In some embodiments, the controller 121 may be a proportional-integral-derivative (PID) controller capable of changing the temperature of the heat sink 114 by a small amount on a relatively short time scale. For example, the controller 121 may change the temperature of the heat sink 114 by about 0.5°C or less, about 0.2°C or less, or even about 0.05°C or less. Thus, the thermal wave may have an amplitude of about 1.0°C or less, about 0.4°C or less, or even about 0.10°C or less. The controller 121 may be capable of changing the temperature of the heat sink 114 via the temperature modification device 118 from one set point to another and back to form a thermal wave having a frequency from about 0.001 to about 0.5 Hz, such as from about 0.005 to about 0.1 Hz, or from about 0.01 to about 0.05 Hz. In some embodiments, the controller 121, the temperature modification device 118, and the heat sink 114 may together produce a thermal wave having a variable frequency. Based on a measurement from the temperature sensor 116 (if present), a known input to the temperature modification device 118, or other means, properties of the thermal wave may be known (e.g., a phase, amplitude, frequency at a specific time, rate of frequency change, *etc.*).

The device 100 further includes a flow cell 122 configured to pass a liquid 124 over the polymer material 112 of the substrate 110. The flow cell 122 may define a void 126 adjacent the polymer material 112 of the substrate 110, as well as an inlet 128 and an outlet 130 through which the liquid 124 may flow. An O-ring 131 or another appropriate sealing mechanism may retain the liquid 124 within the flow cell 122 adjacent the polymer material 112 over the substrate 110.

The liquid 124 may include an analyte 132 that specifically binds to the polymer material 112 and change thermal properties thereof, as described above. For example, the analyte 132 may include one or more strains of bacteria. The analyte 132 (which may include multiple analytes 132a and 132b) specifically binds to the polymer material 112 and changes thermal properties thereof, as described above. If multiple analytes 132a and 132b are present in the liquid 124, the analytes 132a, 132b may have similar functionalities, such that each of the analytes 132a, 132b bind to the polymer material 112. The analytes 132a, 132b may bind to the polymer material 112 with different affinities. In some embodiments, the first analyte 132a may include living bacteria, and the second analyte 132b may include dead bacteria of the same species. In other embodiments, the first analyte 132a may include bacteria, and the second analyte 132b may include an analogue bacteria.

A temperature sensor 134 (*e.g.,* a thermocouple or another device) provided according to the invention and configured to detect a temperature of the liquid 124 in (e.g., flowing through) the flow cell 122. The computer 120 may record the temperature of the liquid 124 by, for example, measuring a resistance of the temperature sensor 134 via the controller 121 and/or the processor 123, and correlating that resistance to a temperature. The temperature of the liquid 124 may be different from the temperature of the heat sink 114, and may vary based at least in part on the presence or absence of the analyte 132 and its concentration in the liquid 124. For example, temperature resistance of substrates based on the concentration of analytes is described in U.S. Patent Application Publication 2014/0011198 A1, "Heat-Transfer Resistance Based Analysis Bioparticles," published January 9, 2014.

According to the invention, the processor 123 is configured to calculate a concentration of the analyte 132 in the liquid 124 based at least in part on a phase shift between the thermal wave produced by the heat sink 114 and an attenuated thermal wave in the liquid 124 after the thermal wave passes through the substrate 110 and the polymer material 112.

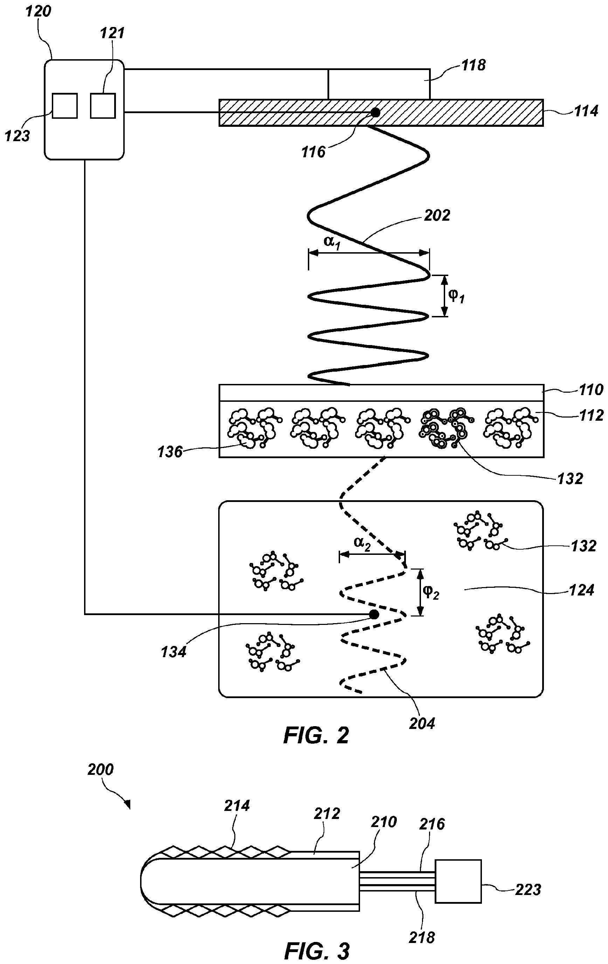

FIG. 2 is a simplified schematic representation showing how the thermal wave may travel in the device 100 of FIG. 1. FIG. 2 includes some of the components shown in FIG. 1, but shows them separated to allow representation of thermal waves traveling through and between the components. In particular, FIG. 2 shows the heat sink 114 thermally coupled to the temperature modification device 118 and the temperature sensor 116, which are connected to the computer 120. The concentration of the analyte 132 may be measured based on the differences between the thermal wave at the heat sink 114 and the thermal wave in the liquid 124, without a separate calibration step.

The heat sink 114 may produce a thermal wave 202 and transfer the thermal wave 202 to the substrate 110 and the polymer material 112 thereon. For example, if the heat sink 114 is initially maintained at a constant temperature of 37°C, the thermal wave 202 may be produced by heating the heat sink 114 to a temperature of 37.1°C and then cooling the heat sink 114 to a temperature of 36.9°C. The heating and cooling of the heat sink 114, driven by the temperature modification device 118, may cause the substrate 110 and the polymer material 112 to heat and cool in a corresponding manner. The thermal wave 202 may have an amplitude $\alpha_1$ and a frequency $\varphi_1$. The amplitude $\alpha_1$ and/or the frequency $\varphi_1$ may vary with time. For example, the thermal wave 202 may have a continuously varying frequency $\varphi_1$.

As discussed above, the presence or absence of the analyte 132 on the substrate 110 may change the thermal conductivity, thermal diffusivity, heat capacity, or another property of the polymer material 112. FIG. 2 illustrates conceptually that the polymer material 112 may define cavities 136 therein adapted to interact with at least a portion of the analyte 132. Without being bound to any particular theory, the cavities 136 may be configured to act to specifically bind the analyte 132. Thus, the polymer material 112 may receive particles or molecules of the analyte 132 from the liquid 124 in some of the cavities 136, based on the concentration of the analyte 132 in the liquid 124. The liquid 124 and the polymer material 112 may reach equilibrium at a given temperature, such that the analyte 132 binds to and separates from the polymer material 112 at equal rates. The thermal properties of the polymer material 112 may depend in part on the fraction of the cavities 136 bound to particles or molecules of the analyte 132.

The substrate 110 and/or the polymer material 112 thereon may alter the thermal wave 202 passing therethrough to form an attenuated thermal wave 204. The attenuated thermal wave 204 may be detected by the temperature sensor 134, and recorded by the computer 120. The attenuated thermal wave 204 may have an amplitude $\alpha_2$ and a frequency $\varphi_2$, which may be different from the amplitude $\alpha_1$ and a frequency $\varphi_1$ of the thermal wave 202. The differences in the amplitudes $\alpha_1$, $\alpha_2$ and/or the frequencies $\varphi_1$, $\varphi_2$ may be correlated to the amount of the analyte 132 bound to the polymer material 112, and thus, to the concentration of the analyte 132 in the liquid 124. Measurement of the differences in the amplitudes $\alpha_1$, $\alpha_2$ and/or the frequencies $\varphi_1$, $\varphi_2$ may allow the device 100 to detect relatively lower amounts of the analyte 132 bound to the polymer material 112 (corresponding to lower concentrations of the analyte 132 in the liquid 124) as compared with conventional methods of measuring the temperature of the liquid 124 at steady state.

In certain embodiments, the analyte 132 may bind to a non-planar surface. For example, FIG. 3 is a simplified schematic diagram showing another device 200 for detecting the analyte 132. The device 200 may include a thermocouple 210 having a base material 212 formed over a surface thereof. For example, the base material 212 may be formed over a generally cylindrical surface of the thermocouple 210, such that an entire end of the thermocouple 210 is enclosed. The thermocouple 210 may include a junction between two materials formulated to provide a temperature-dependent voltage between electrical contacts 216, 218. In some embodiments, the thermocouple 210 may include one or more of a metal *(e.g.,* platinum, gold, iridium, palladium, *etc.)* or an alloy (e.g., a nickel alloy, a copper alloy, a rhodium alloy, a rhenium alloy, an iron alloy, a molybdenum alloy, *etc.).*

The base material 212 may be a polymer material such as polylactic-(L)-acid, which may be referred to in the art as PLLA. PLLA is transparent, inexpensive to produce from environmentally renewable sources (e.g., starch or sugar-containing agricultural products), biodegradable, and biocompatible. Furthermore, PLLA can be solubilized in chloroform to enable application to the thermocouple 210. Another material, rather than PLLA, may be selected to be the base material 212, based on desired properties. In some embodiments, the base material 212 may include polyurethane, polylactic acid, polycaprolactone, poly(lactic-co-glycolic acid), poly(D,L-lactide-co-glycolide), or another selected polymer. The base material 212 may be in the form of a thin, smooth, and homogeneous coating over the exterior of the thermocouple 210. Uniformity of the coating by base material 212 may enable to the device 200 to yield reproducible results. The thickness of the base material 212 may be selected in view of the thermal resistance of the base material 212 to affect the rate at which heat may flow toward or away from the thermocouple 210. Thus, a thinner base material 212 may be beneficial for applications in which a fast response is desired or temperature differentials are small.

The base material 212 may be selected to exhibit at least some elasticity, such that the device 200 may be flexible to allow bending of the thermocouple 210 without breaking the base material 212. This may enable the device 200 to be used for applications requiring tight clearance or bends (*e.g., in vivo* use in catheters).

An assay polymer 214 may be on a surface of the base material 212. In some embodiments, the assay polymer 214 may be directly bonded to the surface of the thermocouple 210, and the base material 212 may be omitted. The assay polymer 214 includes a material for which a heat transfer property varies responsive to an amount of the analyte bound thereto. For example, the thermal conductivity, thermal diffusivity, heat capacity, or another property of the assay polymer 214 may vary with concentration of the analyte on the surface thereof.

In some embodiments, the assay polymer 214 may include an imprinted polymer (an MIP or SIP), DNA, RNA, proteins, or portions or analogs thereof (e.g., antibodies). The assay polymer 214 may be configured to possess a high affinity for a specific binding partner, so that when such binding partners are contacted with the surface of the thermocouple 210, the molecules bind with the assay polymer 214. In some embodiments, the assay polymer 214 may include at least about seven (7) repeating units, such as ten (10) repeating units or more.

The device 200 includes a processor 223 programmed to calculate an amount of the analyte bound to the assay polymer 214. The processor 223 may calculate a concentration of the analyte in a liquid in contact with the device 200 based at least in part on the amount of the analyte bound to the assay polymer 214. For example, the processor 223 may calculate the amount of the analyte by a method as disclosed in U.S. Patent Application Publication 2014/0011198 A1, "Heat-Transfer Resistance Based Analysis Bioparticles," published January 9, 2014; or U.S. Patent Application Publication 2014/0242605 A1, "Heat-Transfer Resistance Based Analysis of Bioparticles," published August 28, 2014. The processor 223 is used to detect a phase shift between a thermal wave at or emanating from a heat sink and an attenuated thermal wave at the thermocouple 210. The processor 223 may then calculate the concentration of the analyte in the liquid based at least in part on a difference in amplitude between the thermal wave at the heat sink and the attenuated thermal wave at the thermocouple 210.

Returning again to FIG. 1, the polymer material 112 may be formed or otherwise provided over the substrate 110. For example, the polymer material 112 may be screen-printed onto a metal substrate 110. Screen-printing may be performed efficiently and scaled to produce mass quantities, with relatively high uniformity in comparison with other methods. Screen-printing of substrates is described in, for example, U.S. Patent Application Publication 2012/0186999 A1, "Electrochemical Sensor," published July 26.

The heat sink 114 is thermally coupled to the substrate 110 at a surface opposite the polymer material 112. For example, the heat sink 114 may be placed in direct physical contact with the substrate 110 such that heat can flow from the heat sink 114 to the substrate 110 by conduction. In some embodiments, a thermally conductive material (e.g., a polymerizable liquid matrix having a thermally conductive filler) may be placed in physical contact with the heat sink 114 and the substrate 110 to eliminate air gaps between the heat sink 114 and the substrate 110. Similarly, the temperature modification device 118 may be thermally coupled to the heat sink 114 by direct physical contact, through a thermally conductive material, or by other appropriate means.

The controller 121 (e.g., a PID controller) may be electrically connected to the temperature modification device 118 to provide power sufficient to drive the temperature of the heat sink 114, and to cause the temperature modification device 118 to change the temperature of the heat sink 114 to produce the thermal wave 202 (FIG. 2).

The flow cell 122 may be secured adjacent the substrate 110 such that the liquid 124 enters the flow cell 122 through the inlet 128, contacts the polymer material 112, and then leaves the flow cell 122 through the outlet 130. In some embodiments, the flow cell 122 may be connected to the heat sink 114 by one or more fasteners 138 (e.g., screws). In other embodiments, the flow cell 122 may be connected to the heat sink 114 by integral threads or by a slip-fit joint. The O-ring 131 or other seal may be configured to keep the liquid 124 from contacting the heat sink 114, the temperature modification device 118, or the back side of the substrate 110 directly.

The temperature sensor 134 may be disposed within the void 126 of the flow cell 122 to measure the temperature of the liquid 124 flowing through the flow cell 122. The temperature sensor 134 may be secured to the flow cell 122 by an adhesive or other appropriate means. The temperature sensor 134 may be electrically connected to the processor 123, which may include an ohmmeter. The processor 123 may be configured to continuously detect the temperature at the temperature sensor 134, and to calculate the concentration of the analyte 132 in the liquid 124 based at least in part on a phase shift between the thermal wave 202 (FIG. 2) produced by the heat sink 114 and the attenuated thermal wave 204 (FIG. 2) in the liquid 124.

The device 100 shown in FIG. 1 and described above may be used to detect any selected analyte 132, such as bacteria. For example, the device 100 may be used for detecting, sensing, and quantifying biological analytes or other chemicals in the liquid 124. The device 100 may be used for detecting, sensing, and quantifying particular strains of bacteria, whether bacteria are living or dead, or discriminating types of bacteria in a complex mixture. The analyte 132 may be a gas, liquid, or solid dissolved or otherwise mixed with the liquid 124. For example, the device 100 may be used for detecting, sensing, quantifying analytes, antibodies, antigens, nucleic acids, *(e.g.,* DNA, RNA, *etc.),* including nucleic acids with particular sequences *(e.g.,* SNPs), proteins, small molecules *(e.g.,* dopamine, histamine, *etc.)* or other substances. In some embodiments, the device 100 may be used for detecting histamine, dopamine, serotonin, adrenalin, methylphenidate, *etc.*

One of the many attractive features of molecular imprinting methods as disclosed herein is that methods can be applied to a diverse range of analytes. The imprinting of small, organic molecules (*e.g.*, pharmaceuticals, pesticides, amino acids and peptides, nucleotide bases, steroids, sugars, *etc.)* is described in, for example, K. Haupt and K. Mosbach, "Molecularly Imprinted Polymers and Their Use in Biomimetic Sensors," Chem. Rev. 100, 2495-2504 (2000); and G. Mustafa and P. Lieberzeit, "MIP Sensors on the Way to Real-World Applications," in Springer Series on Chemical Sensors and Biosensors, vol. 12, pp. 167-187 (Springer, 2012). Somewhat larger organic compounds (e.g., peptides) can also be imprinted via similar approaches. Protocols for imprinting larger structures, such as proteins, cells, and mineral crystals have been proposed in, for example, M. Kempe, M. Glad, and K. Mosbach, "An Approach Towards Surface Imprinting Using the Enzyme Ribonuclease A," J. Molecular Recognition, 8, 35-39 (1995); S. Hjerten et al., "Gels Mimicking Antibodies in Their Selective Recognition of Proteins," Chromatographia 44, 227-234 (1997); H. Shi et al., "Template-Imprinted Nanostructured Surfaces for Protein Recognition," Nature 398, 593-597 (1999); A. Aherne et al. "Bacteria-Mediated Lithography of Polymer Surfaces," J. Am. Chem. Soc. 118, 8771-8772 (1996); and S. M. D'Souza, et al., "Directed Nucleation of Calcite at a Crystal-Imprinted Polymer Surface," Nature 398, 312-316 (1999). Molecular imprinting as a bridge to drug advanced drug delivery is described in B. Sellergren and C. Allender, "Molecularly Imprinted Polymers: A Bridge to Advanced Drug Delivery," Advanced Drug Delivery Reviews 57, 1733-1741 (2005).

To detect the analyte 132, the liquid 124 containing the analyte 132 may be passed through the flow cell 122, adjacent and in contact with the polymer material 112 over the substrate 110. The analyte 132 (e.g., particles, molecules, or bacteria) binds to the polymer material 112, changing one or more thermal properties of the polymer material 112. The liquid 124 may flow continuously through the flow cell 122 during detection, or the flow may terminate before detection begins. The thermal wave 202 (FIG. 2) and the attenuated thermal wave 204 may travel through the liquid 124 whether the liquid 124 is flowing or stagnant. The thermal properties of liquid 124 may differ for flowing and stagnant liquids 124, but can be determined based on flow properties. In some embodiments, the flow cell 122 and the liquid 124 therein may be brought to a test temperature before detection of the analyte 132. As discussed above, the polymer material 112 may be a molecularly imprinted polymer formulated to bind a particular analyte 132 of interest.

The thermal wave 202 (FIG. 2) is provided from the heat sink 114 to the polymer material 112 through the substrate 110. The controller 121 (e.g., a PID controller) may change the temperature of the heat sink 114 via the temperature modification device 118, such as by raising the temperature and lowering the temperature of the heat sink 114 by a preselected amount and at a preselected frequency. The change in the temperature of the heat sink 114 may be small enough that the change does not interfere significantly with other measurements that may occur simultaneously. For example, the average temperature of the liquid 124 in the flow cell 122 may be measured even though the temperature of the heat sink 114 is varying, so long as the time scale of the average temperature measurement is longer than the frequency of the variation and/or the amount of the temperature variation is small in comparison with the temperature change induced by the interaction of the analyte 132 with the polymer material 112. In some embodiments, the heat sink 114 may provide a thermal wave 202 having a frequency from about 0.001 to about 0.5 Hz, such as from about 0.005 to about 0.1 Hz, or from about 0.01 to about 0.05 Hz. Furthermore, the frequency of the thermal wave 202 may vary during testing (e.g., the frequency may be continuously varied from a low frequency to a high frequency or vice versa). The thermal wave 202 may have an amplitude of about 1.0°C or less, about 0.4°C or less, or even about 0.10°C or less.

The temperature of the liquid 124 in the flow cell 122 may be tested, and the result may be compared with the temperature of the heat sink 114.

The concentration of the analyte 132 in the liquid 124 is calculated at least in part on a phase shift between the thermal wave 202 produced by the heat sink 114 and the attenuated thermal wave 204 wave in the liquid 124. A comparison of the thermal wave 202 and the attenuated thermal wave 204 may be performed by the processor 123 based on responses of liquids of known concentration. In some embodiments, the comparison of the thermal wave 202 with the attenuated thermal wave 204 may be based at least in part on the amplitudes the phase shift, or another property.

Measurement of the thermal wave enables measurement of thermal resistance without significantly changing the overall temperature of the polymer material 112. Without being bound to any particular theory, such a measurement appears to be a thermal analog to the measurement of capacitance or inductance in the field of electronics. For example, measuring resistance reveals some information about an electronic device or material, but measuring capacitance or impedance reveals additional information, such as how the device or material responds to a load. Similarly, measuring thermal resistance by the methods disclosed herein can reveal additional information that measuring a steady-state temperature difference cannot.

For example, when applying a thermal wave, different types of information are available in the form of a change in amplitude, frequency and/or phase of the attenuated thermal wave in the liquid upon binding of a target to the receptor. The phase shift may vary based on the frequency of the input. The amount of information provided by a thermal wave is much greater than steady-state analysis, and the information may enable detection or differentiation of a wider variety of materials.

Furthermore, and again without being bound to any particular theory, an increase in thermal mass of the polymer material 112 may occur upon binding of the analyte 132 onto its receptor (i.e., the cavities 136). Before binding of the analyte 132, the cavities 136 may be filled with liquid. Upon binding of the analyte 132 into its receptor, the liquid may be replaced by the analyte 132, thus increasing the thermal mass of the entire transducer system.

In some embodiments, the first analyte 132a may be distinguished from the second analyte 132b by removing the second analyte 132b from the polymer material 112. For example, if the first analyte 132a is living bacteria, and the second analyte 132b is dead bacteria, the dead bacteria may be washed or rinsed from polymer material 112 (e.g., with a buffer), leaving the living bacteria behind. Differences in affinity between the first analyte 132a and the second analyte 132b may facilitate such discrimination. In some embodiments, the first analyte 132a may be the template molecule used to form the polymer material 112, and the second analyte 132b may be a molecule or bacteria having some similar functionality. Therefore the second analyte 132b may bind, at least weakly, to the polymer material 112.

EXAMPLES

Examples 1 through 5 build on aspects of biosensing devices described generally in U.S. Patent Application Publication 2014/0011198 A1, "Heat-Transfer Resistance Based Analysis Bioparticles," published January 9, 2014. They do not form part of the present invention, but may be used as background knowledge or in conjunction with the present invention.

Example 1: Preparation of MIP having a template for detecting dopamine.

Ethylene glycol dimethacrylate (EGDM), methacrylic acid (MAA), dopamine hydrochloride salt (99%), and methanol were purchased from Acros Organics (Loughborough, United Kingdom). Prior to polymerization, the stabilizers in the MAA and EGDM were removed by filtration over alumina. 4,4'-azobis(4-cyanovaleric acid) and serotonin creatinine sulfate monohydrate (98%) were purchased from Sigma-Aldrich (Gillingham, United Kingdom). For the heat-transfer measurements, a 1x phosphate buffered saline (PBS) solution was prepared with Dulbecco tablets obtained from Oxoid Limited (Basingstoke, United Kingdom).

A mixture of MAA (0.54 g, 6.6 mmol), EGDM (2.96 g, 14.9 mmol), and 4,4'-azobis(4-cyanovaleric acid) (65 mg) was dissolved in methanol (3.67 ml) and water (0.57 ml) together with dopamine (0.063 g, 0.33 mmol), the template molecule. This mixture was degassed with $N_2$ and heated to initiate polymerization. To allow full completion of the reaction, the mixture was kept at 65°C for 12 hours. After polymerization, the bulk polymer was ground and sieved to obtain microparticles having diameters smaller than 10 $\mu$m. Dopamine was removed from the MIP powders by continuous extraction with a 50/50 mixture of methanol and water. After 6 hours, the MIP was substantially free of dopamine, as verified by AT-IR spectroscopy with a NICOLET™ 380 FT-IR device from Thermo Scientific (Loughborough, United Kingdom). Subsequently, the MIP powder was dried in an oven for 12 hours at 100°C. A non-imprinted polymer (NIP) was synthesized as a control according to the same method, but without the presence of the dopamine.

Example 2: Testing of MIP for detecting dopamine

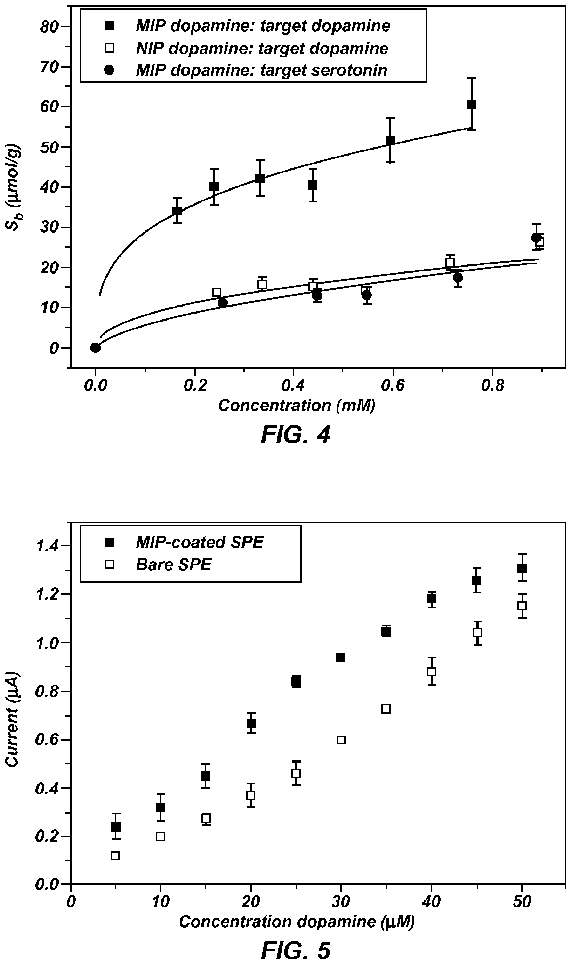

Specificity and binding isotherms of the MIP and NIP particles were determined by optical batch rebinding experiments with an Agilent 8453 spectrophotometer (Stockport, United Kingdom). For the rebinding experiments, 20 mg of MIP or NIP powder was added to 5 ml of aqueous dopamine solutions in concentrations between 0.3 to 1.0 mM. The resulting suspensions were shaken for 12 hours on a rocking table at room temperature. Subsequently, the suspensions were filtered and the free concentration of dopamine ($C_f$) was determined by UV-vis spectroscopy. The bound concentrations ($S_b$) of dopamine were calculated per gram of MIP and NIP and binding isotherms, and are shown in FIG. 4. By fitting the binding isotherms, the specificity of the MIP toward the template dopamine was determined. To test the selectivity, the competitor molecule serotonin was used, since its structure is very similar to dopamine. For these experiments, 20 mg of MIP powder was added to 5 ml of aqueous serotonin solutions and binding isotherms were determined after filtration of the suspensions.

FIG. 4 shows that there is a significant difference in binding between the MIP and its reference, the NIP. To determine the specificity, the imprint factor (IF) was used, which is the amount bound to the MIP divided by the amount bound to the reference NIP at a selected concentration. The binding isotherms were fitted with a two-parameter fit of the following type to analyze the imprint factor at a specific concentration (Equation 1):

Equation 1: $$S_b = A \cdot C_f^v$$

Equation 1 corresponds to the Freundlich isotherm and may be used for fitting of MIP binding isotherms if the distribution of the binding sites and affinity constants are assumed to be heterogeneous. At Cf = 0.3 mM, the IF was 3.1 $\pm$ 0.1, whereas higher concentrations yielded slightly lower IF values (-2.5) due to saturation of the binding sites. The results were comparable to other dopamine MIPs in literature. The response of the MIP to the competitor serotonin was not significantly different than the reference, demonstrating the selectivity of the system.

Example 3: Preparation of MIP-coated Screen-Printed Electrodes (SPEs)

Experiments carried out throughout the following Examples utilize Screen-Printed Electrodes (SPEs) (41 mm x 7 mm), which comprise a three-electrode configuration with a 3-mm graphite working electrode, a graphite counter electrode and an Ag/AgCl reference electrode.

SPEs were fabricated with stencil designs to form a 3-mm diameter working electrode, using a screen-printing machine (MicroDEK 1760RS, available from DEK, Weymouth, UK). First, a carbon-graphite ink formulation (C2000802P2, available from Gwent Electronic Materials Ltd, UK) was printed onto a polyester substrate having a thickness of 250 $\mu$m. The carbon-graphite ink was cured in a fan-oven at 60°C for 30 minutes. A dielectric paste (D2070423D5, available from Gwent Electronic Materials Ltd) was printed onto the polyester substrate to cover the connections. The dielectric paste was cured at 60°C for 30 minutes. The reproducibility of this batch of sensors was found to correspond to less than 4% RSD toward a redox probe, $[Ru(NH_3)]^{2+/3+}$/0.1 M KCl, using an edge connector.

The MIPs were incorporated into the ink of the SPEs on the basis of the weight percent of $M_P$ and $M_I$, where $M_P$ is the mass of particulate and $M_I$ is the mass of the ink formulation used in the printing process. For the purposes of these Examples, the weight percent of $M_P$ was 30% and the weight percent of $M_I$ was 70%.

Example 4: Cyclic voltammetry measurements of SPEs

Cyclic voltammetric measurements were carried out using a potentiostat (Autolab PG-STAT, available from Metrohm, Utrecht, The Netherlands), using three electrodes. Graphitic screen-printed electrodes and MIP-coated SPEs as described in Example 3 were used as the defined working electrodes. A platinum counter and a saturated calomel electrode (SCE) as the reference electrode complete the circuit. This electroanalytical protocol was studied over a range of dopamine concentrations from 0 to 50 $\mu$M, in steps of 5 $\mu$M, within a nitrogen-degassed pH-7.4 phosphate-buffered saline (PBS) solution. The oxidation peak at +0.20 V was used as the analytical parameter. This experimental procedure was carried out over the potential range from -0.2 V to +0.8 V at a scan rate of 50 mV/sec. The resulting calibration curves are shown in FIG. 5. Analysis of the oxidation peak height versus dopamine concentration shows that the response in both electrodes was approximately linear.

The response of both electrodes to dopamine can be represented with a linear fit ($R^2 = 0.97$), indicating the sensitive regime of the sensor platform. For the bare SPEs, the gradient was 0.023 $\mu$A/$\mu$M dopamine, while for the MIP-modified SPE the gradient was 0.025 $\mu$A/$\mu$M dopamine. The limit of detection was defined as the concentration at which the signal is three times the standard deviation. The limit of detection was 4.7 $\pm$0.05 $\mu$M for the MIP-coated SPE and 4.0 $\pm$ 0.06 $\mu$M for the bare SPE.

Example 5: Heat-Transfer Method (HTM)

A flow cell having an inside diameter of 6 mm and a height of 4 mm, with a total interior volume of 110 $\mu$l, was made of acrylic (available under the trademark PERSPEX®, from Lucite International, of Lancashire, United Kingdom). The flow cell was coupled to the potentiostat system described in Example 4, and was sealed with an O-ring. The contact area between the flow cell and the potentiostat system was 28 mm$^2$. The MIP-coated SPEs (described in Example 3) were mounted horizontally and pressed mechanically onto a copper block, which served as a heat sink. The temperature $T_1$ of the copper block was actively controlled by a proportional-integral-derivative (PID) controller with control parameters P = 8, I = 1, and D = 0, and measured by a thermocouple. The temperature $T_1$ of the copper block was maintained at 37.00°C.

A second thermocouple was positioned above the surface of the MIP-coated SPEs, which measured the temperature $T_2$ in the liquid. The thermal resistance, abbreviated as $R_{th}$ (°C/W), was determined by dividing the temperature difference ($T_1$-$T_2$) by the input power P (in Watts) consumed while keeping the temperature constant at 37.00°C (Equation 2).

$$\text{Equation 2: } R_{th} = \frac{T_1 - T_2}{P}.$$

The MIP-coated SPEs were stabilized in phosphate-buffered saline (PBS) solutions, and then increasing concentrations of dopamine (0 to 900 nM) in the solution were added to the flow cell. After stabilization of the signal, the $R_{th}$ values at each concentration were determined. Corresponding dose-response curves were constructed, and are shown in FIG. 4.

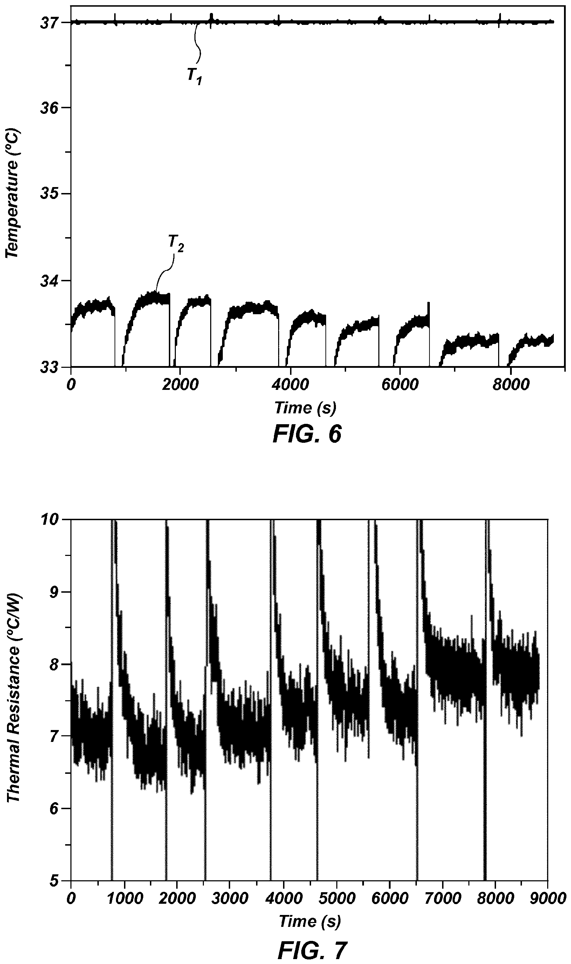

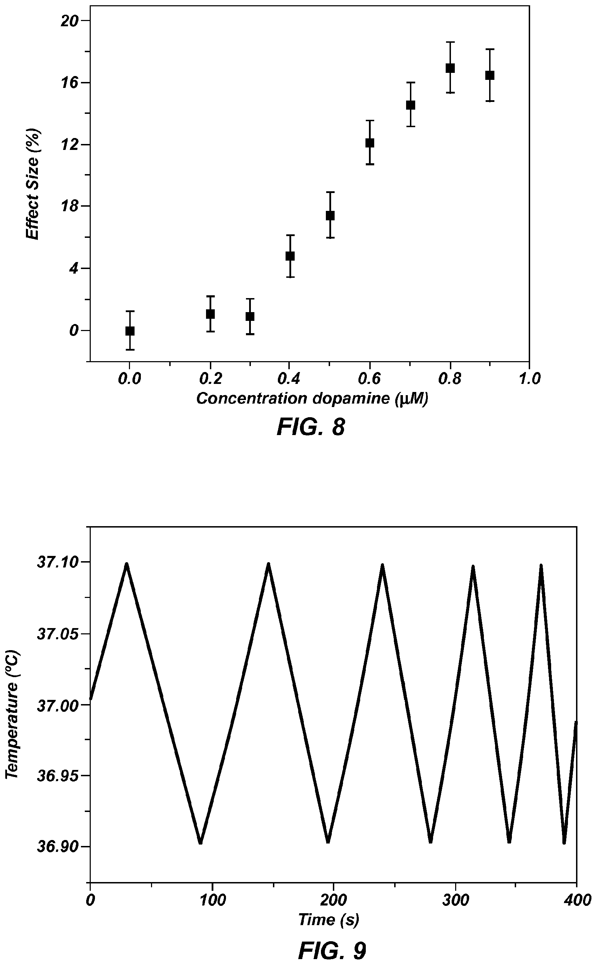

The flow cell was placed in an environment with a stable ambient temperature of 20.00 $\pm$ 0.02°C. The temperature of the copper block, $T_1$, was strictly controlled at 37.00 $\pm$ 0.02°C by a PID controller. The flow cell was filled with pure PBS solution; after stabilization of $T_2$, increasing concentrations of dopamine in PBS solutions were added (0 to 1000 nM). As shown in FIG. 6, a change in the concentration of the solution flowing into the flow cell resulted in a quick drop in $T_2$. After reaching a stable plateau level, the sensor cell was left to stabilize for at least 15 minutes. The decrease in $T_2$ can then be solely attributed to the binding of the target molecules to the MIP layer. FIG. 7 shows the time-dependent thermal resistance values, and FIG. 8 shows the corresponding $R_{th}$ data in the form of a dose-response curve. The normalized values shown in FIG. 8 were calculated by dividing $R_{th}$ observed after each addition to the base-line signal.

FIG. 7 illustrates that the thermal resistance $R_{th}$ increased stepwise from 6.80 $\pm$ 0.10°C/W to 7.92 $\pm$ 0.09°C/W by gradually increasing the dopamine concentration to 900 nM in PBS. This corresponds to a percentage increase of 16.5%, significantly higher than the noise on the signal (1.1%), indicating that the effect is due to binding of the target to the nanocavities of the MIP. When the same test was performed on the reference NIP electrode, the thermal resistance did not significantly change with increasing concentrations of dopamine. Thus, the MIP appears to be specific to dopamine.

As shown in the calculated dose-response curve in FIG. 8; at concentrations up to 800 nM, the binding effect increased linearly with the concentration. At higher concentrations, a trend toward saturation was exhibited, which may be attributed to increasing occupation of the binding sites. With a linear fit, the limit of detection was determined to be $350 \pm 30$ nM, which is a significant improvement compared to cyclic voltammetry (having a limit of detection of $4700 \pm 50$ nM, see Example 4).

Example 6: Thermal Wave Transport Analysis (TWTA)

Besides analyzing the heat-transport through the functionalized chip, the phase shift in response to the heat sink was studied simultaneously on the same sample as on which the HTM (Example 5) was performed.

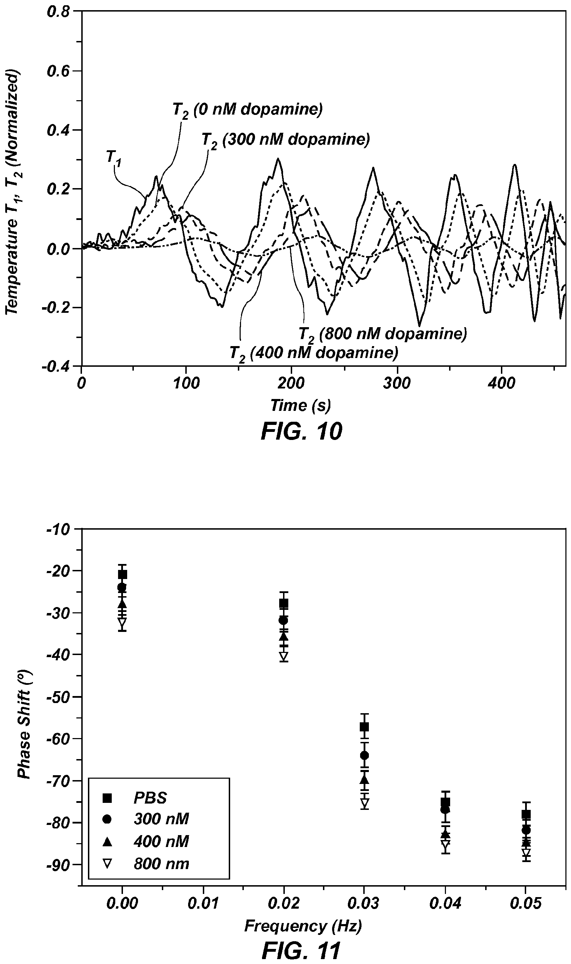

At four chosen dopamine concentrations in PBS (0, 300 nM, 400 nM, and 800 nM) the PID controller transmitted a thermal wave through the heat sink by a $22-\Omega$ radial leaded high-power resistor (Type MPR Series, available from TE Connectivity, of Schaffhausen, Switzerland) through a thermally conductive silicone paste (SILCOTHERM SG502, available from ACC Silicones Ltd., of Somerset, UK). The thermal wave had an amplitude of 0.1°C and variable frequency from 0.01 Hz to 0.05 Hz, as shown in FIG. 9. When dopamine was bound to the MIP particles, a delay in the phase ($\varphi_1 \neq (\varphi_2)$ and a reduction in amplitude ($\alpha_1 \neq \alpha_2$) of the thermal wave output were measured at $T_2$, as shown in FIG. 10. Because the thermal wave had an amplitude of only 0.1 °C and was applied at no more than four distinct points it time, the thermal wave did not affect the stability of the system or the thermal resistance values calculated.

In FIG. 10, the phase shift observed between the input thermal wave ($T_1$) and resulting wave passing through the MIP-coated SPE exposed to a pure PBS buffer solution was due to the time required to transfer heat from the heat sink to the center of the liquid compartment. A slight increase of the phase shift, accompanied with a decrease of the amplitude of the signal, was observed when the MIP-coated SPE was exposed to a 300 nM solution of dopamine in PBS. With higher concentrations of dopamine, the measured phase shift increased more and the amplitude decreased more. Without being bound to any particular theory, it appears that binding of the neurotransmitter to the MIP-layer resulted in a rise in the heat-transfer resistance at the solid-liquid interface. This leads to slower dissipation of the heat from the heat sink to the liquid compartment and appears to explain the results observed in FIG. 10.

FIG. 11 shows the observed phase as a function of the frequency of the applied thermal wave. As shown in FIG. 11, a large change in the phase shift appears between 0.02 Hz and 0.03 Hz, with smaller changes between 0 Hz and 0.02 Hz and between 0.03 Hz and 0.05 Hz. Thus, a frequency of 0.03 Hz was selected to measure target-receptor dynamics in subsequent Examples. At concentrations above 300 nM, a significant effect in the thermal wave output was measured at 0.03 Hz. At this frequency, a phase shift of $-57° \pm 1°$ was observed in PBS, while at 800 nM this increased to $-75° \pm 2°$, corresponding to a $31\% \pm 2\%$ percent increase.

As shown in FIG. 8, the detection limit for dopamine by the heat transfer method (HTM, Example 5) was about 350 mN. However, measuring the phase-shift response, as described in Example 6, dopamine was successfully measured at 300 nM. At a higher concentration of 800 nM, the heat transfer method produced an effect of $16 \pm 1\%$, which is nearly a factor of two lower than for the phase-shift response. Thus, the Thermal Wave Transfer Analysis (TWTA, Example 6) can improve detection of dopamine.

Example 7: Detection of dopamine in bananas

Bananas were ground for 4 min in a combined steamer and blender (Avent model SCF870/20, available from Royal Philips, of Eindhoven, The Netherlands) and subsequently centrifuged at 3200 rpm for 5 minutes. The supernatant was filtered to obtain a clear liquid, which was spiked with increasing concentrations of dopamine (62.5, 125, 250, 500, 1000, 2000 nM). At concentrations of 500 nM and higher, a significant effect on the thermal resistance was observed.

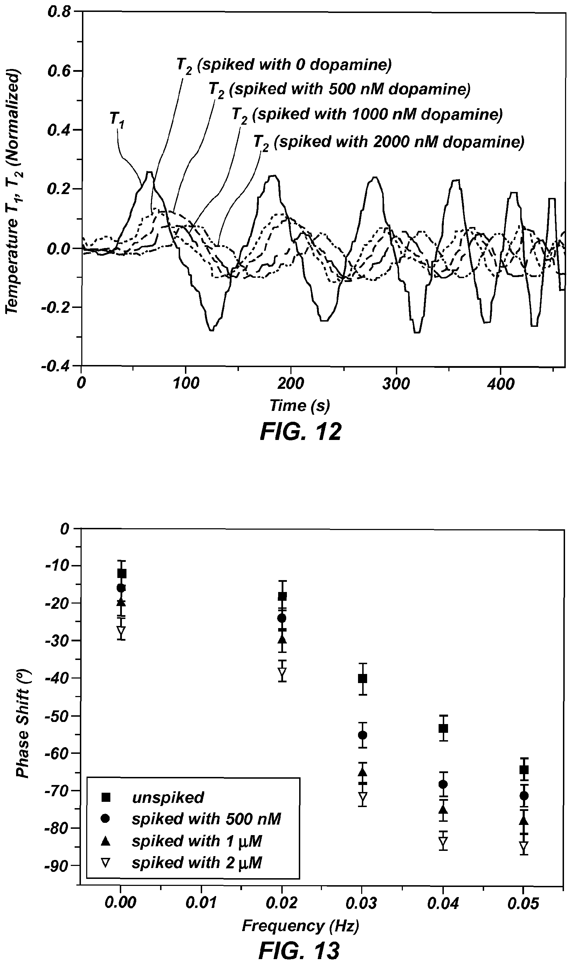

The test described in Example 6 was repeated using the banana-derived liquid spiked with dopamine. The result of the thermal wave outputs normalized to the initial temperature of 37.00°C and corresponding phase shifts are shown in FIG. 12. Only the results for 500 nM and higher concentrations are provided because at lower concentrations no significant difference was observed. A gentle filter (10 point median) was applied to the data to correct for viscosity effects. FIG. 13 shows the observed phase as a function of the frequency of the applied thermal wave. At the spiked concentration of 500 nM, a phase shift of $-55 \pm 3$ Hz was measured compared to $37 \pm 2$ Hz in a pure, non-spiked solution. In percentage increase, a difference of $46\% \pm 2\%$ was measured, which is a combination of the effect of the spiked dopamine concentration and of the initial dopamine present in the banana. Because 500 nM is still in the concentration range in which dopamine is present in biological samples, this Example 7 shows that the Thermal Wave Transfer Analysis (TWTA) technique may be used to measure biologically relevant dopamine concentrations.

Conventional methods are difficult to implement to measure food-related samples because of the high viscosity and the presence of other interfering compounds in food samples, such as large proteins. For example, the limit of detection of certain compounds may increase due to non-specific binding and higher noise levels (compare Example 6, wherein concentrations of 300 nM in buffer were detectable, with Example 7, wherein concentrations of 500 nM were detectable in spiked banana fluid).

Table 1 below compares the detection limits for MIP-modified SPEs of dopamine in buffer solutions and in a food sample. Table 1 shows that thermal methods can provide advantages over conventional electrochemical methods because the limit of detection in buffer solutions is approximately an order of magnitude lower. Furthermore, thermal methods enable measurement of complex food samples. Compared to HTM, analyzing the transport of thermal waves had a significantly higher effect size (31% vs 16% at 800 nM in dopamine buffer solutions) and enhanced the detection limit by requiring less stringent temperature control.

Table 1: Detection limits of MIP-modified SPEs of dopamine

| Detection technique | Detection limit of buffer solutions (nM) | Detection limit of food sample spiked with dopamine (nM) |
| --- | --- | --- |
| Cyclic voltammetry | 4700 $\pm$ 50 (Example 4) | - |
| Heat-transfer method (HTM) | 350 $\pm$ 30 (Example 5) | -500 nM (Example 7) |
| Thermal wave transport analysis (TWTA) | 300 $\pm$ 35 (Example 6) | -500 nM (Example 7) |

The direct mixing of MIP particles with screen-printing ink may eliminate some steps in preparation of electrodes, and may enable mass-production of functionalized electrodes. Thermal wave transport analysis (TWTA) may result in limits of detection for dopamine in the nanomolar regime for not only buffer solutions, but also with a relevant food sample. An additional benefit is that this technique can be performed simultaneously with the heat-transfer method, allowing direct validation of the results. The described methodology offers a new approach for fast and cost-effective detection of neurotransmitters, which may be used in the fields of biomedical and clinical research.

The methods and devices described herein may be used in conjunction with steady-state or thermal-wave analysis techniques. Various shapes of substrates may be used, and data (e.g., temperature) may be collected at various points, such as in the liquid to be analyzed, in a substrate coated with polymer material, or in a coated thermocouple.

Methods described herein may be used to provide real-time or nearly real-time characterization of bacteria that is conventionally performed in laboratories having complex equipment and highly trained personnel. Thus, the methods and devices may enable faster and cheaper data collection, and may enable improved outcomes by, for example, identifying bacterial outbreaks within a population. Such methods may be beneficial in health care, environmental and food safety (e.g., by detecting water-, air-, and food-borne bacteria), and counter-terrorism *(e.g.,* by detecting anthrax, *etc.).*

## Claims

1. A device (100) for detecting an analyte (132), the device comprising:

   a substrate (110) having a polymer material (112) formed on a surface thereof, the polymer material formulated to bind to the analyte, wherein a heat transfer property of the polymer material is formulated to vary based on an amount of the analyte bound thereto;
   a heat transfer element (114) thermally coupled to a surface of the substrate opposite the polymer material;
   a temperature modification device (118) thermally coupled to the heat transfer element; further **characterized by**
   a controller (121) configured to cause the temperature modification device to produce a thermal wave (202) emanating from the heat transfer element;
   a flow cell (122) located and configured to pass a liquid (124) over the polymer material of the substrate;
   a temperature sensor (134) configured to detect a temperature ($T_2$) of the liquid passing over the polymer material; and
   a processor (123) configured to calculate a concentration of an analyte (132) in the liquid based at least in part on a phase shift between the thermal wave at the heat transfer element and an attenuated thermal wave (204) in the liquid.

**2.** The device of claim 1, wherein the processor (123) is configured to calculate the concentration of the analyte based at least in part on a difference in amplitude between the thermal wave at the heat transfer element (114) and the attenuated thermal wave in the liquid (124).

**3.** The device of claim 1, wherein the controller (123) is configured to change a temperature of the heat transfer element (114) at a variable frequency.

**4.** The device of claim 1, wherein the polymer material (112) comprises an imprinted polymer, such as a molecularly imprinted polymer or a surface-imprinted polymer.

**5.** The device of claim 1, wherein the polymer material (112) comprises a material selected from the group consisting of DNA, RNA, proteins, and portions and analogs thereof.

**6.** The device of claim 1, wherein the polymer material (112) is formulated to bind to a first bacteria with a first affinity higher than a second affinity of the polymer material to a second bacteria.

**7.** The device of claim 6, wherein the first bacteria comprises living bacteria, and wherein the second bacteria comprises dead bacteria, the living bacteria and the dead bacteria being of the same species or wherein the first bacteria comprises a first species, and wherein the second bacteria comprises a second species, the second species being an analogue of the first species.

**8.** A method for detecting an analyte, the method comprising:

passing a liquid (124) containing an analyte (132) over a polymer material (112) on a substrate (110), the polymer material formulated to bind to the analyte, wherein a heat transfer property of the polymer material is formulated to vary based on an amount of the analyte bound thereto;
binding the analyte to the polymer material; further **characterized by** the steps of
providing a thermal wave (202) from a heat transfer element (114) to the polymer material through the substrate;
detecting a temperature ($T_2$) of the liquid; and
calculating a concentration of the analyte in the liquid based at least in part on a phase shift between the thermal wave produced by the heat transfer element and an attenuated thermal (204) wave in the liquid.

**9.** The method of claim 8, further comprising generating the thermal wave with a controller (121) configured to change a temperature ($T_1$) of a temperature modification device (118) thermally coupled to the heat transfer element.

**10.** The method of claim 8, wherein calculating a concentration of the analyte (132) in the liquid (124) comprises determining a difference in amplitude between the thermal wave at the heat transfer element (114) and the attenuated thermal wave in the liquid (124).

**11.** The method of claim 8, wherein providing a thermal wave from a heat transfer element (114) to the polymer material (112) through the substrate (110) comprises changing a frequency of the thermal wave.

**12.** The method of claim 8, wherein detecting a temperature of the liquid (124) comprises detecting the temperature of the liquid as a function of time.

**13.** The method of claim 8, wherein calculating a concentration of the analyte (132) in the liquid (124) comprises calculating a concentration of a biological analyte in the liquid.

**14.** The method of claim 13, wherein calculating a concentration of a biological analyte in the liquid (124) comprises calculating a concentration of histamine in the liquid.

**15.** The method of claim 8, wherein passing a liquid (124) containing an analyte (132) over a polymer material (112) on a substrate (110) comprises passing the liquid containing the analyte over a molecularly imprinted polymer or wherein passing a liquid containing an analyte over a polymer material on a substrate comprises passing the liquid containing the analyte over a material selected from the group consisting of DNA, RNA, proteins, and portions and analogs thereof.

**16.** The method of claim 8, wherein providing a thermal wave from a heat transfer element (114) to the polymer material

(112) through the substrate (110) comprises changing a temperature ($T_1$) of the heat transfer element (114) by less than 0.2 °C.

17. The method of claim 8, wherein calculating a concentration of the analyte (132) in the liquid (124) comprises calculating a concentration of bacteria in a mixture comprising a plurality of species.

18. The method of claim 8, wherein calculating a concentration of the analyte (132) in the liquid (124) comprises calculating a concentration of living bacteria in a mixture comprising living and dead bacteria of the same species.

19. The method of claim 8, further comprising washing the polymer material (112) to remove material other than the analyte therefrom.

20. A method of forming a device (100) for detecting an analyte (132) as defined in any one or more of the claims 1-7, the method comprising:

forming a polymer material (112) over a surface of a substrate (110), the polymer material formulated to bind to the analyte, wherein a heat transfer property of the polymer material is formulated to vary based on an amount of the analyte bound thereto;
thermally coupling a heat transfer element (114) to a surface of the substrate opposite the polymer material;
thermally coupling a temperature modification device (118) to the heat transfer element; further **characterized by** the steps of
configuring a controller (120) to cause the temperature modification device to produce a thermal wave (202) emanating from the heat transfer element;
configuring a flow cell (122) to pass a liquid (124) over the polymer material of the substrate;
configuring a temperature sensor (134) to detect a temperature ($T_2$) of the liquid passing over the polymer material; and
configuring a processor (123) to calculate a concentration of an analyte (132) in the liquid based at least in part on a phase shift between the thermal wave at the heat transfer element and an attenuated thermal wave (204) in the liquid.

21. The method of claim 20, wherein forming a polymer material (112) over a surface of a substrate (110) comprises screen-printing the polymer material over the surface of the substrate or wherein forming a polymer material over a surface of a substrate comprises forming a molecularly imprinted polymer over the surface of the substrate.

**Patentansprüche**

1. Vorrichtung (100) zum Nachweisen eines Analyten (132), wobei die Vorrichtung Folgendes umfasst:

ein Substrat (110) mit einem Polymermaterial (112), das auf einer Oberfläche davon ausgebildet ist, wobei das Polymermaterial dazu formuliert ist, an den Analyten zu binden, wobei eine Wärmeübertragungseigenschaft des Polymermaterials dazu formuliert ist, basierend auf einer Menge des daran gebundenen Analyten zu variieren;
ein Wärmeübertragungselement (114), das thermisch mit einer dem Polymermaterial gegenüberliegenden Oberfläche des Substrats gekoppelt ist;
eine Temperaturmodifikationsvorrichtung (118), die thermisch mit dem Wärmeübertragungselement gekoppelt ist; ferner **gekennzeichnet durch**
eine Steuerung (121), dazu konfiguriert, zu bewirken, dass die Temperaturmodifikationsvorrichtung eine thermische Welle (202) erzeugt, die von dem Wärmeübertragungselement ausgeht;
eine Strömungszelle (122), dazu angeordnet und konfiguriert, eine Flüssigkeit (124) über das Polymermaterial des Substrats zu leiten;
einen Temperatursensor (134), dazu konfiguriert, eine Temperatur ($T_2$) der über das Polymermaterial strömenden Flüssigkeit zu erfassen; und
einen Prozessor (123), dazu konfiguriert, eine Konzentration eines Analyten (132) in der Flüssigkeit zumindest teilweise basierend auf einer Phasenverschiebung zwischen der thermischen Welle am Wärmeübertragungselement und einer abgedämpften thermischen Welle (204) in der Flüssigkeit zu berechnen.

2. Vorrichtung nach Anspruch 1, wobei der Prozessor (123) dazu konfiguriert ist, die Konzentration des Analyten zumindest teilweise basierend auf einer Amplitudendifferenz zwischen der thermischen Welle am Wärmeübertragungselement (114) und der abgedämpften thermischen Welle in der Flüssigkeit (124) zu berechnen.

3. Vorrichtung nach Anspruch 1, wobei die Steuerung (123) dazu konfiguriert ist, eine Temperatur des Wärmeübertragungselements (114) mit einer variablen Frequenz zu ändern.

4. Vorrichtung nach Anspruch 1, wobei das Polymermaterial (112) ein geprägtes Polymer umfasst, wie beispielsweise ein molekular geprägtes Polymer oder ein oberflächengeprägtes Polymer.

5. Vorrichtung nach Anspruch 1, wobei das Polymermaterial (112) ein Material umfasst, ausgewählt aus der Gruppe bestehend aus DNA, RNA, Proteinen und Teilen und Analoga davon.

6. Vorrichtung nach Anspruch 1, wobei das Polymermaterial (112) dazu formuliert ist, an ein erstes Bakterium mit einer ersten Affinität zu binden, die höher ist als eine zweite Affinität des Polymermaterials zu einem zweiten Bakterium.

7. Vorrichtung nach Anspruch 6, wobei die ersten Bakterien lebende Bakterien umfassen, und wobei die zweiten Bakterien tote Bakterien umfassen, wobei die lebenden Bakterien und die toten Bakterien von derselben Spezies sind oder wobei die ersten Bakterien eine erste Spezies umfassen, und wobei die zweiten Bakterien eine zweite Spezies umfassen, wobei die zweite Spezies ein Analogon der ersten Spezies ist.

8. Verfahren zum Nachweisen eines Analyten, wobei das Verfahren Folgendes umfasst:

Leiten einer Flüssigkeit (124), die einen Analyten (132) enthält, über ein Polymermaterial (112) auf einem Substrat (110), wobei das Polymermaterial dazu formuliert ist, an den Analyten zu binden, wobei eine Wärmeübertragungseigenschaft des Polymermaterials dazu formuliert ist, basierend auf einer Menge des daran gebundenen Analyten zu variieren;
Binden des Analyten an das Polymermaterial; ferner **gekennzeichnet durch** die Schritte:

Bereitstellen einer thermischen Welle (202) von einem Wärmeübertragungselement (114) zu dem Polymermaterial durch das Substrat;
Erfassen einer Temperatur ($T_2$) der Flüssigkeit; und
Berechnen einer Konzentration des Analyten in der Flüssigkeit zumindest teilweise basierend auf einer Phasenverschiebung zwischen der durch das Wärmeübertragungselement erzeugten thermischen Welle und einer abgedämpften thermischen Welle (204) in der Flüssigkeit.

9. Verfahren nach Anspruch 8, ferner umfassend das Erzeugen der thermischen Welle mit einer Steuerung (121), dazu konfiguriert, eine Temperatur ($T_1$) einer mit dem Wärmeübertragungselement thermisch gekoppelten Temperaturmodifikationsvorrichtung (118) zu ändern.

10. Verfahren nach Anspruch 8, wobei das Berechnen einer Konzentration des Analyten (132) in der Flüssigkeit (124) das Bestimmen einer Amplitudendifferenz zwischen der thermischen Welle am Wärmeübertragungselement (114) und der abgedämpften thermischen Welle in der Flüssigkeit (124) umfasst.

11. Verfahren nach Anspruch 8, wobei das Bereitstellen einer thermischen Welle von einem Wärmeübertragungselement (114) zu dem Polymermaterial (112) durch das Substrat (110) das Ändern einer Frequenz der thermischen Welle umfasst.

12. Verfahren nach Anspruch 8, wobei das Erfassen einer Temperatur der Flüssigkeit (124) das Erfassen der Temperatur der Flüssigkeit als Funktion der Zeit umfasst.

13. Verfahren nach Anspruch 8, wobei das Berechnen einer Konzentration des Analyten (132) in der Flüssigkeit (124) das Berechnen einer Konzentration eines biologischen Analyten in der Flüssigkeit umfasst.

14. Verfahren nach Anspruch 13, wobei das Berechnen einer Konzentration des biologischen Analyten in der Flüssigkeit (124) das Berechnen einer Konzentration von Histamin in der Flüssigkeit umfasst.

15. Verfahren nach Anspruch 8, wobei das Leiten einer Flüssigkeit (124), die einen Analyten (132) enthält, über ein Polymermaterial (112) auf einem Substrat (110) umfasst, dass die Flüssigkeit, die den Analyten enthält, über ein molekular geprägtes Polymer geleitet wird, oder wobei das Leiten einer Flüssigkeit, die einen Analyten enthält, über einem Polymermaterial auf einem Substrat umfasst, dass die Flüssigkeit, die den Analyten enthält, über ein Material geleitet wird, das ausgewählt ist aus der Gruppe bestehend aus DNA, RNA, Proteinen und Teilen und Analoga davon.

16. Verfahren nach Anspruch 8, wobei das Bereitstellen einer thermischen Welle von einem Wärmeübertragungselement (114) zu dem Polymermaterial (112) durch das Substrat (110) das Ändern einer Temperatur ($T_1$) des Wärmeübertragungselements (114) um weniger als 0,2 °C umfasst.

17. Verfahren nach Anspruch 8, wobei das Berechnen einer Konzentration des Analyten (132) in der Flüssigkeit (124) das Berechnen einer Bakterienkonzentration in einem Gemisch umfasst, das mehrere Spezies umfasst.

18. Verfahren nach Anspruch 8, wobei das Berechnen einer Konzentration des Analyten (132) in der Flüssigkeit (124) das Berechnen einer Konzentration von lebenden Bakterien in einer Mischung umfasst, welche lebende und tote Bakterien derselben Spezies umfasst.

19. Verfahren nach Anspruch 8, ferner umfassend das Waschen des Polymermaterials (112), um anderes Material als den Analyten daraus zu entfernen.

20. Verfahren zum Ausbilden einer Vorrichtung (100) zum Nachweisen eines Analyten (132) wie in einem oder mehreren der Ansprüche 1-7 definiert, wobei das Verfahren Folgendes umfasst:

Ausbilden eines ein Polymermaterials (112), über einer Oberfläche eines Substrats (110), wobei das Polymermaterial dazu formuliert ist, an den Analyten zu binden, wobei eine Wärmeübertragungseigenschaft des Polymermaterials dazu formuliert ist, basierend auf einer Menge des daran gebundenen Analyten zu variieren;
thermisches Koppeln eines Wärmeübertragungselements (114) mit einer dem Polymermaterial gegenüberliegenden Oberfläche des Substrats;
thermisches Koppeln einer Temperaturmodifikationsvorrichtung (118) mit dem Wärmeübertragungselement; ferner **gekennzeichnet durch** die Schritte:

Konfigurieren einer Steuerung (120) dazu, zu bewirken, dass die Temperaturmodifikationsvorrichtung eine thermische Welle (202) erzeugt, die von dem Wärmeübertragungselement ausgeht;
Konfigurieren einer Strömungszelle (122) dazu, eine Flüssigkeit (124) über das Polymermaterial des Substrats zu leiten;
Konfigurieren eines Temperatursensors (134) dazu, eine Temperatur ($T_2$) der über das Polymermaterial strömenden Flüssigkeit zu erfassen; und
Konfigurieren eines Prozessors (123) dazu, eine Konzentration eines Analyten (132) in der Flüssigkeit zumindest teilweise basierend auf einer Phasenverschiebung zwischen der thermischen Welle am Wärmeübertragungselement und einer abgedämpften thermischen Welle (204) in der Flüssigkeit zu berechnen.

21. Verfahren nach Anspruch 20, wobei das Ausbilden eines Polymermaterials (112) über einer Oberfläche eines Substrats (110) das Siebdrucken des Polymermaterials über der Oberfläche des Substrats umfasst oder wobei das Ausbilden eines Polymermaterials über einer Oberfläche eines Substrats das Ausbilden eines molekular geprägten Polymers über der Oberfläche des Substrats umfasst.

**Revendications**

1. Dispositif (100) de détection d'un analyte (132), le dispositif comprenant :

un substrat (110) ayant un matériau polymère (112) formé sur une surface de celui-ci, le matériau polymère étant formulé pour se lier à l'analyte, dans lequel une propriété de transfert de chaleur du matériau polymère est formulée pour varier sur la base d'une quantité de l'analyte lié à celui-ci ;
un élément de transfert de chaleur (114) couplé thermiquement à une surface du substrat opposée au matériau polymère ;
un dispositif de modification de température (118) couplé thermiquement à l'élément de transfert de chaleur ;
**caractérisé en outre par**
un dispositif de commande (121) configuré pour amener le dispositif de modification de température à produire une onde thermique (202) émanant de l'élément de transfert de chaleur ;

une cellule d'écoulement (122) située et configurée pour faire passer un liquide (124) sur le matériau polymère du substrat ;

un capteur de température (134) configuré pour détecter une température ($T_2$) du liquide passant sur le matériau polymère ; et

un processeur (123) configuré pour calculer une concentration d'un analyte (132) dans le liquide sur la base au moins en partie d'un déphasage entre l'onde thermique au niveau de l'élément de transfert de chaleur et une onde thermique atténuée (204) dans le liquide.

2. Dispositif de la revendication 1, dans lequel le processeur (123) est configuré pour calculer la concentration de l'analyte sur la base au moins en partie d'une différence d'amplitude entre l'onde thermique au niveau de l'élément de transfert de chaleur (114) et l'onde thermique atténuée dans le liquide (124).

3. Dispositif de la revendication 1, dans lequel le dispositif de commande (123) est configuré pour modifier une température de l'élément de transfert de chaleur (114) à une fréquence variable.

4. Dispositif de la revendication 1, dans lequel le matériau polymère (112) comprend un polymère à empreinte, tel qu'un polymère à empreinte moléculaire ou un polymère imprimé en surface.

5. Dispositif de la revendication 1, dans lequel le matériau polymère (112) comprend un matériau choisi dans le groupe constitué d'ADN, d'ARN, de protéines et de parties et d'analogues de ceux-ci.

6. Dispositif de la revendication 1, dans lequel le matériau polymère (112) est formulé pour se lier à une première bactérie ayant une première affinité plus élevée qu'une deuxième affinité du matériau polymère par rapport à une deuxième bactérie.

7. Dispositif de la revendication 6, dans lequel la première bactérie comprend une bactérie vivante, et dans lequel la deuxième bactérie comprend une bactérie morte, la bactérie vivante et la bactérie morte étant de la même espèce ou dans lequel la première bactérie comprend une première espèce, et dans lequel la deuxième bactérie comprend une deuxième espèce, la deuxième espèce étant un analogue de la première espèce.

8. Procédé de détection d'un analyte, le procédé comprenant :

le passage d'un liquide (124) contenant un analyte (132) sur un matériau polymère (112) sur un substrat (110), le matériau polymère étant formulé pour se lier à l'analyte, dans lequel une propriété de transfert de chaleur du matériau polymère est formulée pour varier sur la base d'une quantité de l'analyte lié à celui-ci ;

la liaison de l'analyte au matériau polymère : **caractérisé en outre par** les étapes de

fourniture d'une onde thermique (202) d'un élément de transfert de chaleur (114) au matériau polymère à travers le substrat ;

détection d'une température ($T_2$) du liquide ; et

calcul d'une concentration de l'analyte dans le liquide sur la base au moins en partie d'un déphasage entre l'onde thermique produite par l'élément de transfert de chaleur et une onde thermique atténuée (204) dans le liquide.

9. Procédé de la revendication 8, comprenant en outre la génération de l'onde thermique avec un dispositif de commande (121) configuré pour modifier une température ($T_1$) d'un dispositif de modification de température (118) couplé thermiquement à l'élément de transfert de chaleur.

10. Procédé de la revendication 8, dans lequel le calcul d'une concentration de l'analyte (132) dans le liquide (124) comprend la détermination d'une différence d'amplitude entre l'onde thermique au niveau de l'élément de transfert de chaleur (114) et l'onde thermique atténuée dans le liquide (124).

11. Procédé de la revendication 8, dans lequel la fourniture d'une onde thermique d'un élément de transfert de chaleur (114) au matériau polymère (112) à travers le substrat (110) comprend la modification d'une fréquence de l'onde thermique.

12. Procédé de la revendication 8, dans lequel la détection d'une température du liquide (124) comprend la détection de la température du liquide en fonction du temps.

**13.** Procédé de la revendication 8, dans lequel le calcul d'une concentration de l'analyte (132) dans le liquide (124) comprend le calcul d'une concentration d'un analyte biologique dans le liquide.

**14.** Procédé de la revendication 13, dans lequel le calcul d'une concentration d'un analyte biologique dans le liquide (124) comprend le calcul d'une concentration d'histamine dans le liquide.

**15.** Procédé de la revendication 8, dans lequel le passage d'un liquide (124) contenant un analyte (132) sur un matériau polymère (112) sur un substrat (110) comprend le passage du liquide contenant l'analyte sur un polymère à empreinte moléculaire ou dans lequel le passage d'un liquide contenant un analyte sur un matériau polymère sur un substrat comprend le passage du liquide contenant l'analyte sur un matériau choisi dans le groupe constitué d'ADN, d'ARN, de protéines et de parties et d'analogues de ceux-ci.

**16.** Procédé de la revendication 8, dans lequel la fourniture d'une onde thermique d'un élément de transfert de chaleur (114) au matériau polymère (112) à travers le substrat (110) comprend la modification d'une température ($T_1$) de l'élément de transfert de chaleur (114) de moins de 0,2 °C.

**17.** Procédé de la revendication 8, dans lequel le calcul d'une concentration de l'analyte (132) dans le liquide (124) comprend le calcul d'une concentration de bactéries dans un mélange comprenant une pluralité d'espèces.

**18.** Procédé de la revendication 8, dans lequel le calcul d'une concentration de l'analyte (132) dans le liquide (124) comprend le calcul d'une concentration de bactéries vivantes dans un mélange comprenant des bactéries vivantes et mortes de la même espèce.

**19.** Procédé de la revendication 8, comprenant en outre le lavage du matériau polymère (112) pour en enlever le matériau autre que l'analyte.

**20.** Procédé de formation d'un dispositif (100) pour détecter un analyte (132) tel que défini dans l'une quelconque ou plusieurs des revendications 1 à 7, le procédé comprenant :

la formation d'un matériau polymère (112) sur une surface d'un substrat (110), le matériau polymère étant formulé pour se lier à l'analyte, dans lequel une propriété de transfert de chaleur du matériau polymère est formulée pour varier sur la base d'une quantité de l'analyte lié à celui-ci ;
le couplage thermique d'un élément de transfert de chaleur (114) à une surface du substrat opposée au matériau polymère ;
le couplage thermique d'un dispositif de modification de température (118) à l'élément de transfert de chaleur ; **caractérisé en outre par** les étapes de
configuration d'un dispositif de commande (120) pour amener le dispositif de modification de température à produire une onde thermique (202) émanant de l'élément de transfert de chaleur ;
configuration d'une cellule d'écoulement (122) pour faire passer un liquide (124) sur le matériau polymère du substrat ;
configuration d'un capteur de température (134) pour détecter une température ($T_2$) du liquide passant sur le matériau polymère ; et
configuration d'un processeur (123) pour calculer une concentration d'un analyte (132) dans le liquide sur la base au moins en partie d'un déphasage entre l'onde thermique au niveau de l'élément de transfert de chaleur et une onde thermique atténuée (204) dans le liquide.

**21.** Procédé de la revendication 20, dans lequel la formation d'un matériau polymère (112) sur une surface d'un substrat (110) comprend la sérigraphie du matériau polymère sur la surface du substrat ou dans lequel la formation d'un matériau polymère sur une surface d'un substrat comprend la formation d'un polymère à empreinte moléculaire sur la surface du substrat.

**FIG. 1**

**FIG. 2**

**FIG. 3**

*FIG. 4*

*FIG. 5*

**FIG. 6**

**FIG. 7**

FIG. 8

FIG. 9

FIG. 10

FIG. 11

FIG. 12

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140011198 A1
- EP 2772753 A
- US 6161028 A
- JP 2005345385 A
- US 20090281272 A1
- US 20140242605 A1
- US 20120186999 A1

**Non-patent literature cited in the description**

- **BART VAN GRINSVEN.** The Heat-Transfer Method: A Versatile Low-Cost, Label-Free, Fast, and User-Friendly Readout Platform for Biosensor Applications. *ACS APPLIED MATERIALS AND INTERFACES,* 08 August 2014, vol. 6
- **K. HAUPT ; K. MOSBACH.** Molecularly Imprinted Polymers and Their Use in Biomimetic Sensors. *Chem. Rev.,* 2000, vol. 100, 2495-2504
- MIP Sensors on the Way to Real-World Applications. **G. MUSTAFA ; P. LIEBERZEIT.** Springer Series on Chemical Sensors and Biosensors. Springer, 2012, vol. 12, 167-187
- **M. KEMPE ; M. GLAD ; K. MOSBACH.** n Approach Towards Surface Imprinting Using the Enzyme Ribonuclease A. *J. Molecular Recognition,* 1995, vol. 8, 35-39
- **S. HJERTEN et al.** Gels Mimicking Antibodies in Their Selective Recognition of Proteins. *Chromatographia,* 1997, vol. 44, 227-234
- **H. SHI et al.** Template-Imprinted Nanostructured Surfaces for Protein Recognition. *Nature,* 1999, vol. 398, 593-597
- **A. AHERNE et al.** Bacteria-Mediated Lithography of Polymer Surfaces. *J. Am. Chem. Soc.,* 1996, vol. 118, 8771-8772
- **S. M. D'SOUZA et al.** Directed Nucleation of Calcite at a Crystal-Imprinted Polymer Surface. *Nature,* 1999, vol. 398, 312-316
- **B. SELLERGREN ; C. ALLENDER.** Molecularly Imprinted Polymers: A Bridge to Advanced Drug Delivery. *Advanced Drug Delivery Reviews,* 2005, vol. 57, 1733-1741